# DEMANDE DE BREVET EUROPEEN

(11) **EP 0 943 691 A1**
(43) Date de publication de la demande: **22.09.1999**
(21) Numéro de dépôt: 99400390.3
(22) Date de dépôt: 18.02.1999
(51) Int. Cl.: C12Q 1/68, C12Q 1/04

(54) **Procédé de diagnostic d'agents pathogènes respiratoires par biologie moleculaire**

(30) Priorité: 19.02.1998 FR 9801998
(71) Demandeur: ETAT FRANCAIS Représenté par le Délégué Général pour l'Armement, 00460 Armées (FR)
(72) Inventeur: Dorchain, Nathalie, 54136 Bouxieres aux Dames (FR)

(57) **Abrégé**

La présente invention concerne un procédé de diagnostic d'agents pathogènes respiratoires à partir d'un prélèvement d'un échantillon clinique sur un patient, comprenant la mise en solution de l'échantillon clinique dans un tube réactionnel, l'extraction de l'ADN de chacun des agents pathogènes, l'amplification de fragments ciblés de ceux-ci à l'aide d'amorces par réactions de polymérisation en chaîne multiples (PCR multiplex) et leur révélation, caractérisé en ce le diagnostic est appliqué à la détection simultanée, dans le même tube réactionnel et à partir d'un seul prélèvement d'au moins deux agents pathogènes choisis parmi *Legionella pneumophila, Mycoplasma pneumoniae* et *Chlamydia pneumoniae* en effectuant au moins une PCR à l'aide d'une combinaison de paires d'amorces constituées par des séquences de nucléotides judicieusement choisies pour chacun des agents pathogènes.

## Description

La présente invention concerne un procédé de diagnostic d'agents pathogènes respiratoires difficiles à diagnostiquer.

Ce procédé permet leur détection simultanée en utilisant une technique d'amplification génique.

Les agents pathogènes respiratoires visés sont *Legionella pneumophila, Mycoplasma pneumoniae* et *Chlamydia pneumoniae.*

On sait que les atteintes aiguës du parenchyme pulmonaire ou pneumopathies sont la première cause de mortalité par maladie infectieuse. Elles touchent 1% des collectivités d'adultes jeunes, 5% des patients hospitalisés et représentent 15 à 20% des infections nosocomiales. Elles sont à l'origine de détresses respiratoires aiguës d'évolution mortelle dans 20 à 60% des cas.

En milieu hospitalier, le diagnostic étiologique précis d'une pneumopathie demeure inconnu dans 30 à 50% des cas, en dépit du recours à de multiples prélèvements et examens, tels que examens cytobactériologiques des crachats, lavages broncho-alvéolaires, brossages distaux protégés, hémocultures, ponctions pleurales.

Ces difficultés diagnostiques sont en partie liées à la présence d'agents pathogènes difficiles ou impossibles à cultiver avec les techniques bactériologiques courantes. Les agents pathogènes tels que *Legionella pneumophila, Mycoplasma pneumoniae* et *Chlamydia pneumoniae* sont à l'origine de plus de 30% des pneumopathies communautaires chez les adultes jeunes.

Legionella pneumophila fut découverte en 1976 aux Etats-Unis d'Amérique, lorsqu'elle atteignit 182 anciens combattants lors du 58ème congrès de "l'American Legion".

*Mycoplasma pneumoniae* est la plus petite forme de vie autonome connue (0,2 à 0,8µm).

*Chlamydia pneumoniae* a été isolé pour la première fois chez un enfant taïwanais souffrant d'une conjonctivite à Seattle. C'est 20 ans plus tard qu'il sera mis en cause lors d'une épidémie de pneumonie chez des étudiants de l'université de Washington.

En pratique médicale courante, la recherche des agents pathogènes précités par des techniques traditionnelles telles que le diagnostic direct, la culture, le diagnostic indirect par la recherche d'anticorps spécifiques ou sérologie, sont aléatoires et de faible sensibilité.
En effet, comme le fait apparaître le tableau ci-dessous, les cultures sont lentes et malaisées à obtenir et la recherche d'anticorps ne peut se faire que deux semaines après l'épisode clinique initial.

| Agents path | Diagnostic direct par cultures | | | Diagnostic indirect:sérologie | | |
|---|---|---|---|---|---|---|
| | délai de réponse | sensibilité | spécificité | délai de réponse | sensibilité | spécificité |
| *L.pneumophila* | 2-15j | moyenne | bonne | 15j | moyenne | bonne |
| *M.pneumoniae* | 6-20j | moyenne | bonne | 15j | moyenne | bonne |
| *C.pneumoniae* | 3j | faible | bonne | 15j | moyenne | bonne |

Les éléments qui président au choix du traitement antibiotique reposent davantage sur l'appréciation du terrain, des antécédents respiratoires infectieux, du caractère communautaire ou nosocomial, de la connaissance de l'épidémiologie bactérienne et des profils de résistance, que sur des aspects cliniques souvent peu contributifs.

Les antibiotiques sont donc attribués en première intention sur des données statistiques et l'évaluation du traitement doit être systématiquement effectuée entre 48 et 72 heures. Cette attitude conduit le plus souvent à associer des antibiotiques coûteux pour parer à toute éventualité. Un tiers de la consommation d'antibiotiques est lié aux maladies broncho-pulmonaires.

Il est donc impératif de disposer de moyens diagnostiques fiables et rapides pour la recherche de ces agents pathogènes dont la fréquence est loin d'être négligeable.

Il est connu que l'utilisation de techniques de biologie moléculaire permet de faire un diagnostic en quelques heures et d'adapter immédiatement le traitement, limitant ainsi les risques d'échec et le coût de l'antibiothérapie.

La biologie moléculaire permet une détection rapide et sensible d'agents infectieux en révélant une partie spécifique de leurs génomes.

Plusieurs publications internationales décrivent des techniques permettant le diagnostic des différents agents pathogènes mentionnés ci-dessus.

C.Y.W. Tong et M.Sillis ont décrit dans J.Clin. Pathol. 1993,46,p.313-317 une réaction de polymérisation en chaîne (PCR) nichée pour la détection de *Chlamydia pneumoniae* et de *Chlamydia psittaci* dans des échantillons pulmonaires. La première amplification est effectuée sur un domaine de 333 paires de bases du gène Omp A codant pour une protéine de la membrane externe de *Chlamydia pneumoniae* et de *Chlamydia psittaci* avec des amorces externes dénommées CP1 et CP2. La seconde amplification est réalisée avec des amorces internes dénommées CPC et CPD sur un domaine de 207 paires de bases du gène Omp A.

Dans Clinical infectious diseases 1993:17; S83-9, B. de Barbeyrac, C. Bernet Poggi, F. Fébrer, H. Renaudin, M. DUPON C. Bébéar ont décrit la détection de *Mycoplasma pneumoniae* et *Mycoplasma genitalum*.

*Mycoplasma pneumoniae* possède la propriété d'adhérer à de nombreux supports tels que le verre, les globules rouges, les cellules d'épithélium trachéal. L'adhésine P1 de *Mycoplasma pneumoniae* a été identifiée comme une protéine de 165 à 190000 daltons. Elle est située à la surface de l'extrémité effilée ou "tip" qui joue un rôle important dans l'adhérence de *Mycoplasma pneumoniae.* Les amorces dénommées MPP11 et MPP12 amplifient un fragment de 466 paires de bases du gène codant pour l'adhésine P1, spécifique de *Mycoplasma pneumoniae.* L'amorce dénommée MPP12 telle que ci-dessus et l'amorce MP-i amplifient un second fragment de 183 paires de bases de ce gène.

M. Kolde et A. Saito ont décrit dans Clinical infectious diseases 1995;21; 199-201 le diagnostic de *Legionella pneumophila.* Cette bactérie est mise en évidence, dans un premier temps, par amplification d'un fragment de 649 paires de bases du gène "mip" (macrophage infectivity potentiator), puis, dans un second temps, par amplification d'un fragment de 489 paires de bases de ce même gène.

Cependant aucune technique de biologie moléculaire permettant de diagnostiquer rapidement, efficacement et simultanément les trois agents pathogènes respiratoires *Legionella pneumophila, Mycoplasma pneumoniae et Chlamydiae pneumoniae* n'existe.

L'invention a pour but d'économiser le coût de réactifs et le temps de technicien.

A cette fin, un diagnostic simultané, c'est-à-dire, à partir d'un seul prélèvement et dans un même tube réactionnel, a été envisagé.

Pour ce faire, la présente invention a pour objet un procédé de diagnostic d'agents pathogènes respiratoires à partir d'un prélèvement d'un échantillon clinique sur un patient, comprenant la mise en solution de l'échantillon clinique dans un tube réactionnel, l'extraction de l'ADN de chacun des agents pathogènes, l'amplification de fragments ciblés de ceux-ci à l'aide d'amorces par réactions de polymérisation en chaîne multiples (PCR multiplex) et leur révélation, caractérisé en ce le diagnostic est appliqué à la détection simultanée, dans le même tube réactionnel et à partir d'un seul prélèvement d'au moins deux agents pathogènes choisis parmi *Legionella pneumophila, Mycoplasma pneumoniae* et *Chlamydiapneumoniae* en effectuant au moins une PCR à l'aide d'une combinaison de paires d'amorces constituées par des séquences de nucléotides choisies de la façon suivante pour chacun des agents pathogènes:
a) pour *Legionella pneumophila:*
   paires d'amorces de séquences comprises entre les bases 1 et 702 du gène dénommé "mip" de cet agent pathogène,
b) pour *Mycoplasma pneumoniae:*
   paires d'amorces de séquences comprises entre les bases 1 et 9691 du gène codant pour l'adhésine P1 de cet agent pathogène,
c) pour *Chlamydia pneumoniae:*
   paires d'amorces de séquences comprises entre les bases 1 et 1180 du gène Omp A codant pour une protéine externe de cet agent pathogène.

Plus particulièrement, selon le procédé de l'invention, on effectue au moins une PCR à l'aide d'une combinaison de paires d'amorces choisies parmi les amorces suivantes pour chacun des agents pathogènes:
a) pour *Legionella pneumophila:* 
amorce LmipL 920 de séquence: GCT ACA GAC AAG GAT AAG
TTG
 
amorce LmipR 1548 de séquence: GTT TTG TAT GAC TTT AAT
TCA
 
amorce LmipL 997 de séquence: TAA TCC GGA AGC AAT GGC
TA
 
amorce LmipR 1466 de séquence: CGG CCA ATA GGT CCG CCA
AC
b) pour *Mycoplasma pneumoniae :* amorce MPP11 de séquence: TGC CAT CAA CCC GCG CTT AAC amorce MPP12 de séquence: CCT TTG CAA CTG CTC ATA GTA amorce MP-i de séquence: CAA ACC GGG CAG ATC ACC TTT
c) pour *Chlamydia pneumoniae :*

amorce CP1 de séquence: TTA CAA GCC TTG CCT GTA GG

amorce CP2 de séquence: GCG ATC CCA AAT GTT TAA GGC

amorce CPC de séquence: TTA TTA ATT GAT GGT ACA ATA

amorce CPD de séquence: ATC TAC GGC AGT AGT ATA GTT.

Lorsqu'il s'agit de détecter simultanément *Legionella pneumophila, Mycoplasma pneumoniae* et *Chlamydia pneumoniae,* on effectue une première PCR à l'aide des amorces CP1, CP2, MPP11, MPP12, LmipL 920, LmipR 1548 suivie d'une seconde PCR à l'aide des amorces CPC, CPD, MP12, MP-i, LmipL 997 et LmipR 1466.

Le procédé selon l'invention peut être limité à la détection de deux des agents pathogènes précités, à savoir, *Legionella pneumophila* et *Mycoplasma pneumoniae,* ou *Legionella* *pneumophila* et *Chlamydia pneumoniae,* ou encore, *Mycoplasma pneumoniae* et *Chlamydiapneumoniae,* en n'utilisant que les paires d'amorces citées ci-dessus pour les agents pathogènes correspondants

Le procédé selon la présente invention permet la détection simultanée de *Legionella pneumophila,* de *Mycoplasma pneumoniae* et de *Chlamydia pneumoniae* grâce à la technique d'amplification génique P.C.R.

Il est connu que le séquençage du génome de virus, de bactéries, de parasites responsables de diverses affections permet, notamment, de désigner des cibles nucléotidiques spécifiques de ces agents pathogènes et d'en effectuer le diagnostic.

La première étape du procédé, selon l'invention, de détection des trois agents pathogènes précités est l'extraction de l'ADN de chacun de ces agents.

L'extraction de l'ADN a lieu, de manière connue, en présence d'un tampon de lyse hypertonique associé à un détergent (triton) et d'une protéinase (la protéinase K très active permettant la lyse des protéines et la libération de l'ADN dans le milieu) et un choc thermique.

Ensuite, il s'agit d'obtenir des fragments d'ADN correspondant à des gènes choisis de chacun des agents pathogènes recherchés.

Les étapes suivantes sont:
- La dénaturation, ou séparation des deux brins d'ADN, par la chaleur.
- L'hybridation avec des paires d'amorces qui permet de sélectionner un fragment d'ADN; les amorces étant constituées par des séquences de nucléotides synthétisées en routine par des appareils automatiques.
- L'élongation qui permet d'amplifier le fragment d'ADN compris entre les deux amorces. Elle est effectuée avec une polymérase, en l'occurrence, la Taq polymérase disponible commercialement.

Pour la détection de *Legionella pneumophila,* le gène retenu est le gène dénommé "mip" cité précédemment et composé de 702 paires de bases. On sélectionne un fragment de ce gène à l'aide d'une paire d'amorces .

Les amorces utilisées sont une combinaison d'une amorce sens et d'une amorce antisens choisies, de préférence, parmi les amorces LmipL 920, LmipR 1548, LmipL 997 et LmipR 1466 dont les séquences sont décrites ci-dessus. Cependant, on doit pouvoir utiliser des amorces composées de séquences de bases situées de part et d'autre des séquences précédemment définies.

Si l'on utilise la paire d'amorces externes LmipL 920 et LmipR 1548, on sélectionne un fragment de 649 paires de bases du gène "mip".

Si l'on utilise la paire d'amorces LmipL 997 et LmipR 1466, on sélectionne un fragment de 489 paires de bases du gène "mip".

Pour la détection de *Mycoplasma pneumoniae,* on a retenu le gène codant pour l'adhésine P1 précédemment cité et composé de 9691 paires de bases. On sélectionne un fragment de ce gène à l'aide d'une paire d'amorces .

Les amorces utilisées sont une combinaison d'une amorce sens et d'une amorce antisens choisies, de préférence, parmi les amorces MPP11, MPP12 et MP-i dont les séquences sont décrites ci-dessus. Cependant, comme précédemment, on doit pouvoir utiliser des amorces composées de séquences de bases situées de part et d'autre des séquences précédemment définies.

Si l'on utilise la paire d'amorces externes MPP11 et MPP12, on sélectionne un fragment de 466 paires de bases du gène codant pour l'adhésine P1.

Si l'on utilise la paire d'amorces MPP12 et MP-i, on sélectionne un fragment de 183 paires de bases du gène codant pour l'adhésine P1.

Pour la détection de *Chlamydia pneumoniae,* on a retenu le gène Omp A codant pour une protéine de la membrane externe de cet agent et composé de 1170 paires de bases. On sélectionne un fragment de ce gène à l'aide d'une paire d'amorces .

Les amorces utilisées sont une combinaison d'une amorce sens et d'une amorce antisens choisies, de préférence, parmi les amorces CP1, CP2, CPC et CPD dont les séquences sont décrites ci-dessus. Cependant, comme précédemment, on doit pouvoir utiliser des amorces composées de séquences de bases situées de part et d'autre des séquences précédemment définies.

Si l'on utilise la paire d'amorces externes CP1 et CP2, on sélectionne un fragment de 333 paires de bases du gène Omp A.

Si l'on utilise la paire d'amorces CPC et CPD, on sélectionne un fragment de 207 paires de bases du gène Omp A.

La dernière étape du procédé selon l'invention est la mise en évidence, ou révélation, de l'ADN amplifié pour chacun des agents pathogènes. Elle est réalisée après migration dans un gel d'agarose contenant un agent intercalant, le bromure d'éthidium(BET). La bande fluorescente obtenue est comparée à celle d'un témoin positif et d'un marqueur de poids moléculaire.

On peut se contenter d'une seule PCR , si l'on dispose d'une révélation de grande sensibilité.

Cependant, une seconde PCR augmente, d'une part, la sensibilité de la technique en permettant de faire apparaître des bandes qui ne sont pas forcément apparues lors de la première PCR et, d'autre part, en améliore la spécificité en faisant disparaître des bandes non spécifiques qui auraient pu apparaître lors de la première PCR.

Un mode de réalisation préféré consiste à effectuer une PCR nichée pour *Legionella pneumophila,* une PCR semi-nichée pour *Mycoplasma pneumoniae* et une PCR nichée pour *Chlamydia pneumoniae*.

Une PCR nichée comprend deux PCR successives dont la première aboutit à l'amplification d'un premier fragment d'ADN grâce à une paire d'amorces externes et la seconde à l'amplification d'une portion du premier fragment amplifié, grâce à une paire d'amorces internes différentes des deux amorces précédentes.

Une PCR semi-nichée comprend deux PCR successives dont la première aboutit à l'amplification d'un premier fragment d'ADN, grâce à une paire d'amorces externes, et la seconde à l'amplification d'une portion du premier fragment amplifié, grâce à une paire d'amorces internes dont l'une est identique à l'une des deux amorces précédentes.

C'est ainsi que pour *Legionella pneumophila,* on utilise préférentiellement les amorces externes LmipL 920 et LmipR 1548 pour la première PCR et les amorces internes LmipL 997 et LmipR 1466 pour la seconde PCR.

Pour *Mycoplasma pneumoniae,* on utilise préférentiellement les amorces externes MPP11 et MPP12 pour la première PCR et les amorces internes MPP12 et MP-i pour la seconde PCR.

Pour *Chlamydia pneumoniae,* on utilise préférentiellement les amorces externes CP1 et CP2 pour la première PCR et les amorces internes CPC et CPD pour la seconde PCR.

D'autres avantages et caractéristiques de la présente invention apparaîtront dans la description ci-après d'un exemple de réalisation.

### EXEMPLE :

### EXTRACTION DE L'ADN:

On effectue sur le patient un prélèvement, de préférence d'origine respiratoire, tel que crachat, frottis de gorge, de nez, aspiration bronchique, prélèvement distal protégé, brosse bronchique, lavage broncho-alvéolaire, de liquide péritonéal, ou encore prélèvement tissulaire, de liquide pleural.

On introduit 500µl du prélèvement dans un microtube.

On centrifuge pendant 20 minutes à 14000g à une température de 4°C, puis on élimine le surnageant à la pompe à vide.

En zone protégée, en l'occurrence, en salle de préparation des réactifs dite "salle des mix", on prépare le tampon de lyse à raison de 100µl par microtube.

La composition du tampon de lyse est la suivante:

| | |
|---|---|
| Tris à 10mM | 10µl |
| EDTA à 1mM | 10µl |
| Détergent de dénomination commerciale X100 | 1µl |
| Protéinase K à 100µg/ml | 5µl |
| Eau ultra pure à 18,6MΩ | 1ml |

On transfert les 100µl de culot repris dans un tube de 0,2ml pour thermocycleur.

On place le tube dans le thermocycleur pour un cycle de lyse dont le programme est le suivant:
30 minutes à 56°C
10 minutes à 95°C
5 minutes à 4°C
5 minutes à 95°C
retour à 4°C.

L'échantillon ainsi obtenu peut être stocké à -20°C avant amplification.

### AMPLIFICATION:

### 1ère PCR:

Elle se compose des amplifications suivantes:

Amplification d'un fragment de 333 paires de bases (pbs) d'une partie du gène OmpA de *Chlamydia pneumoniae* avec les amorces CP1 et CP2.

Amplification d'un fragment de 466pbs du gène codant pour l'adhésine P1 de *Mycoplasma pneumoniae* avec les amorces MPP11 et MPP12.

Amplification d'un fragment de 649pbs du gène "mip" de *Legionella pneumophila.*

Pour chaque patient, un témoin d'inhibition est réalisé. Ce témoin comprend un échantillon du prélèvement du patient associé à un témoin positif, ceci afin de s'affranchir de résultats erronés qui seraient dus à l'inhibition de la Taq polymérase par certains produits, notamment l'hémoglobine.

On prépare le mélange réactionnel suivant en salle des "mix" et dans la glace:

| Réactif | quantité par tube en µl | molarité finale(prélève ment compris) |
|---|---|---|
| Tampon Taq X10 | 5 | Xl |
| MgCl₂ 50mM | 2 | 2mM |
| dNTP lOmM | 1 | 200µm |
| amorce CP1 50µM | 1 | 1000nM |
| amorce CP2 50µM | 1 | 1000nM |
| amorce MPP11 50µM | 0,5 | 500nM |
| amorce MPP12 50µM | 0,5 | 500nM |
| amorce LmipL 920 50µM | 0,5 | 500nM |
| amorce LmipR 1548 50µM | 0,5 | 500nM |
| H₂O qsp 40µl | 30 | |
| Taq 5UI/µl | 0,25 | 1,25U/tube |

Dans un microtube pour thermocycleur, on ajoute 40µl de "mix", sans oublier de réaliser les témoins d'inhibition.

Puis, on prépare un témoin d'inhibition contenant 5µl d'échantillon préalablement traité et 5µl de témoin positif (contenant les 3 agents pathogènes).

On amplifie dans le thermocycleur dont le programme est le suivant:
Dénaturation à 95°C pendant 5 minutes
Hybridation à 55°C pendant 30 secondes
Elongation à 72°C pendant 30 secondes

Dénaturation à 95°C pendant 30 secondes
Hybridation à 55°C pendant 30 secondes X40
Elongation à 72°C pendant 30 secondes

Elongation à 72°C pendant 5 minutes
Stockage à 4°C.

### 2ème PCR:

Elle se compose des amplifications suivantes:

Amplification d'un fragment de 207pbs d'une partie du gène OmpA spécifique de *Chlamydia pneumoniae* avec les amorces CPC et CPD.

Amplification d'un fragment de 183pbs d'une partie du gène codant pour l'adhésine P1 de *Mycoplasma pneumoniae* avec les amorces MPP12 et MP-i.

Amplification d'un fragment de 489pbs du gène "mip" de *Legionella pneumophila*.

On prépare le mélange réactionnel suivant en salle des "mix" et dans la glace:

| Réactif | quantité par tube en µl | molarité finale(prélèvement compris) |
|---|---|---|
| Tampon Taq X10 | 5 | Xl |
| MgCl₂ 50mM | 2 | 2mM |
| dNTP 10mM | 1 | 200um |
| amorce CPC 50µM | 1 | 1000nM |
| amorce CPD 50µM | 1 | 1000nM |
| amorce MPP12 50µM | 0,5 | 500nM |
| amorce MP-i 50µM | 0,5 | 500nM |
| amorce LmipL 997 50µM | 0,5 | 500nM |
| amorce LmipR 1466 50µM | 0,5 | 500nM |
| H₂O qsp 49µl | 37 | |
| Taq 5UI/µl | 0,25 | 1,25U/tube |

Dans un microtube pour thermocycleur, on ajoute 49µl de "mix", sans oublier de réaliser les témoins d'inhibition. Puis, on ajoute 1µl d'échantillon de première PCR.

On amplifie dans le thermocycleur dont le programme est le suivant:
Dénaturation à 94°C pendant 5 minutes
Dénaturation à 94°C pendant 30 secondes
Hybridation à 48°C pendant 30 secondes X30
Elongation à 72°C pendant 30 secondes

Elongation à 72°C pendant 5 minutes
Stockage à 4°C.

En outre, le procédé selon l'invention permet de détecter la présence d'un agent pathogène associé aux trois agents pathogènes précités, tel que *Pneumocystis carinii* ou *Bordetella pertussis.*

## Revendications

1. Procédé de diagnostic d'agents pathogènes respiratoires à partir d'un prélèvement d'un échantillon clinique sur un patient, comprenant la mise en solution de l'échantillon clinique dans un tube réactionnel, l'extraction de l'ADN de chacun des agents pathogènes, l'amplification de fragments ciblés de ceux-ci à l'aide d'amorces par réactions de polymérisation en chaîne multiples (PCR multiplex) et leur révélation, caractérisé en ce le diagnostic est appliqué à la détection simultanée, dans le même tube réactionnel et à partir d'un seul prélèvement d'au moins deux agents pathogènes choisis parmi *Legionella pneumophila, Mycoplasma pneumoniae* et *Chlamydia pneumoniae* en effectuant au moins une PCR à l'aide d'une combinaison de paires d'amorces constituées par des séquences de nucléotides choisies de la façon suivante pour chacun des agents pathogènes:
a) pour *Legionella pneumophila:*
paires d'amorces de séquences comprises entre les bases 1 et 702 du gène dénommé "mip" de cet agent pathogène,
b) pour *Mycoplasma pneumoniae:*
paires d'amorces de séquences comprises entre les bases 1 et 9691 du gène codant pour l'adhésine P1 de cet agent pathogène,
c) pour *Chlamydia pneumoniae:*
paires d'amorces de séquences comprises entre les bases 1 et 1170 du gène Omp A codant pour une protéine externe de cet agent pathogène.

2. Procédé selon la revendication 1, caractérisé en ce que l'on effectue au moins une PCR à l'aide d'une combinaison de paires d'amorces choisies parmi les amorces suivantes pour chacun des agents pathogènes:
a) pour *Legionella pneumophila:* 
amorce LmipL 920 de séquence: GCT ACA GAC AAG GAT AAG
TTG
 
amorce LmipR 1548 de séquence: GTT TTG TAT GAC TTT AAT
TCA
 
amorce LmipL 997 de séquence: TAA TCC GGA AGC AAT GGC
TA
 
amorce LmipR 1466 de séquence: CGG CCA ATA GGT CCG CCA
AC
b) pour *Mycoplasma pneumoniae :* amorce MPP11 de séquence: TGC CAT CAA CCC GCG CTT AAC amorce MPP12 de séquence: CCT TTG CAA CTG CTC ATA GTA amorce MP-i de séquence: CAA ACC GGG CAG ATC ACC TTT
c) pour *Chlamydia pneumoniae:*

amorce CP1 de séquence: TTA CAA GCC TTG CCT GTA GG

amorce CP2 de séquence: GCG ATC CCA AAT GTT TAA GGC

amorce CPC de séquence: TTA TTA ATT GAT GGT ACA ATA

amorce CPD de séquence: ATC TAC GGC AGT AGT ATA GTT

3. Procédé selon la revendication 1, caractérisé en ce que l'on effectue deux PCR successives.

4. Procédé selon la revendication 1, caractérisé en ce qu'il est utilisé pour diagnostiquer simultanément *Legionella pneumophila, Mycoplasma pneumoniae* et *Chlamydia pneumoniae*.

5. Procédé selon la revendication 4, caractérisé en ce que l'on effectue une première PCR à l'aide des amorces CP1, CP2, MPP11, MPP12, LmipL 920, LmipR 1548 suivie d'une seconde PCR à l'aide des amorces CPC, CPD, MP12, MP-i, LmipL 997 et LmipR 1466.

6. Procédé selon la revendication 1, caractérisé en ce qu'il est utilisé pour diagnostiquer simultanément *Legionella pneumophila* et *Mycoplasma pneumoniae*.

7. Procédé selon la revendication 1, caractérisé en ce qu'il est utilisé pour diagnostiquer simultanément *Legionella pneumophila* et *Chlamydia pneumoniae.*

8. Procédé selon la revendication 1, caractérisé en ce qu'il est utilisé pour diagnostiquer simultanément *Mycoplasma pneumoniae* et *Chlamydia pneumoniae.*
